# FASCICULE DE BREVET EUROPEEN

(11) **EP 1 212 313 B1**
(45) Date de publication et mention de la délivrance du brevet: **28.05.2003**
(21) Numéro de dépôt: 01974441.6
(22) Date de dépôt: 05.10.2001
(51) Int. Cl.: C07D 311/30, C07D 311/36, C07F 9/655, A61K 31/352, A61P 29/00

(54) **DERIVES DE 7-CARBOXY-FLAVONES, PROCEDE DE PREPARATION ET APPLICATION EN THERAPEUTIQUE**
7-CARBOXYLATISCHE FLAVON DERIVATE, VERFAHREN ZUR HERSTELLUNG UND VERWENDUNG ALS HEILMITTEL
7-CARBOXY-FLAVONE DERIVATIVES, PREPARATION METHOD AND THERAPEUTIC USE

(30) Priorité: 06.10.2000 FR 0012846
(43) Date de publication de la demande: 12.06.2002
(73) Titulaire: Negma-Lerads, 78117 Toussus-Le-Noble (FR)
(72) Inventeur: GESSON, Jean-Pierre, F-86360 MONTAMISE (FR); FONTENEAU, Nadia, F-16230 VILLEBOIS-LAVALETTE (FR); MONDON, Martine, F-86000 POITIERS (FR); CHARBIT, Suzy, F-94000 CRETEIL (FR); FICHEUX, Hervé, F-94130 NOGENT-SUR-MARNE (FR); SCHUTZE, François, F-78860 SAINT-NOM-LA-BRETECHE (FR)
(74) Mandataire: L'Helgoualch, Jean
(86) Numéro de dépôt international: FR0103075
(87) Numéro de publication internationale: WO02028851

(56) Documents cités:
- CHEMICAL ABSTRACTS, vol. 128, no. 22, 1998 Columbus, Ohio, US; abstract no. 274942d, ANA MARIA SILVAN: "EFFECTS OF COMPOUNDS EXTRACTED FROM TANACETUM MICROPHYLLUM" page 974; colonne 1; XP002170500 & PLANTA MED., vol. 64, no. 3, 1998, pages 200-3, ENGL
- CHEMICAL ABSTRACTS, vol. 119, no. 17, 25 octobre 1993 (1993-10-25) Columbus, Ohio, US; abstract no. 173825b, ABAD,M. ET AL.: "ANTI-INFLAMMATORY ACTIVITY OF TWO FLAVONOIDS FROM TANCETUM MICROPHYLLUM." page 48; XP002056027 & J. NAT. PROD., vol. 56, no. 7, 1993, pages 1164-7,

## Description

La présente invention concerne de nouveaux dérivés de flavones et d'isoflavones utiles en thérapeutique, et plus particulièrement de nouveaux dérivés de flavones portant un groupement acide, leur application en thérapeutique pour le traitement de maladies rhumatismales, ainsi qu'un procédé pour leur préparation.

Un grand nombre de dérivés de flavones diversement substitués ont été décrits dans la littérature, ainsi que leurs propriétés pharmacologiques utiles dans de nombreuses applications thérapeutiques, comme par exemple le traitement de l'asthme, de maladies inflammatoires, d'affections gastro-intestinales, d'allergies ou de certaines tumeurs cancéreuses.

Par exemple, le brevet US 5 399 584 décrit des dérivés de flavones utilisables pour la protection de la paroi du tractus gastro-intestinal en complément d'un traitement au moyen d'anti-inflammatoires non stéroïdiens. Le brevet EP 290 915 décrit des acides flavone-3 carboxyliques présentés comme possédant une activité pharmacologique inhibitrice de la formation de radicaux oxygène dans les cellules, permettant d'envisager leur utilisation comme médicaments anti-inflammatoires. Des acides flavones-4' carboxyliques substitués en position 7 par un groupe amide ou sulfonamide, utiles dans le traitement des neuropathies diabétiques, sont décrits dans le brevet FR 2 543 140. Le brevet EP 237 986 décrit d'autres dérivés de flavones substitués sur le noyau phényle par un groupe acide ou ester carboxylique et présentant des propriétés antitumorales.

Certaines carboxy-flavones susceptibles de présenter une activité utile dans le traitement de l'asthme ont été décrites par M.E. Zwangstra et al., J. Med. Chemistry, (1998) 41, 1428-1438. D'autres dérivés de flavones, tels que par exemple, des acides flavone-6 carboxyliques, en particulier la 6-carboxy-2'-isopropoxy-flavone présentant des propriétés anti-allergisantes ainsi qu'une activité spasmolytique, sont décrits dans le brevet US 4 157 334. Le brevet FR 2 689 127 décrit des dit-butyl-3',5'-4'-hydroxy-flavones susceptibles d'être utilisées pour le traitement de dyslipidémies, d'athérosclérose et de cardiopathies ischémiques. La centaureidine, ou 5,7,3'-trihydroxy-3,6,4'-triméthoxyflavone, est un flavonoïde isolé de Tanacetum microphyllum décrit par M.J. Abad et al., J. Nat. Prod. Vol.56, n° 7, pp. 1164-1173 (1993), présentant des propriétés anti-inflammatoires comparables à celle de la phénylbutazone, vérifiées expérimentalement sur la souris. La même activité anti-inflammatoire a été vérifiée sur le métabolisme de l'acide arachidonique (libération de prostaglandine-E₂ et leucotriène-C₄) et décrite dans Planta Med. Vol.64, pp. 200-203 (1998). Cependant, aucun effet inhibiteur des cytokines impliquées dans le processus inflammatoire des maladies rhumatismales n'a été observé.

Dans le domaine des médicaments destinés au traitement de certaines pathologies telles que l'arthrose, des dérivés de rhéine, en particulier la diacérhéine, ont été décrits comme étant particulièrement efficaces à forte dose. Cependant, l'un des inconvénients liés à l'utilisation de ces composés est qu'ils peuvent présenter, chez certains sujets, des effets secondaires gênants en fonction des doses utilisées, et notamment un effet laxatif. Il est donc souhaitable de pouvoir disposer de médicaments anti-inflammatoires possédant une efficacité thérapeutique au moins égale, et dépourvus des effets secondaires inhérents aux rhéines.

La présente invention a pour objet de nouveaux dérivés de flavones, et plus particulièrement des dérivés de flavones et d'isoflavones portant un groupement acide en position 7, présentant d'intéressantes propriétés anti-inflammatoires, permettant leur utilisation comme médicaments pour le traitement de certaines maladies rhumatismales telles que l'arthrose ou la polyarthrite rhumatoïde.

L'invention a également pour objet un procédé de préparation de dérivés de flavones et d'isoflavones portant un groupement acide en position 7, à partir de produits aisément accessibles ou disponibles dans le commerce.

Enfin, l'invention a pour objet l'application en thérapeutique des nouveaux dérivés de flavones et d'isoflavones portant un groupement acide en position 7, plus particulièrement pour le traitement de maladies rhumatismales en thérapeutique humaine et vétérinaire, ainsi que l'utilisation desdits dérivés de flavones et d'isoflavones pour la préparation d'un médicament pour le traitement des maladies rhumatismales telles que l'arthrose ou la polyarthrite rhumatoïde.

Les nouveaux dérivés de flavones et d'isoflavones conformes à la présente invention peuvent être représentés par les formules générales (Ia) et (Ib) ci-après : 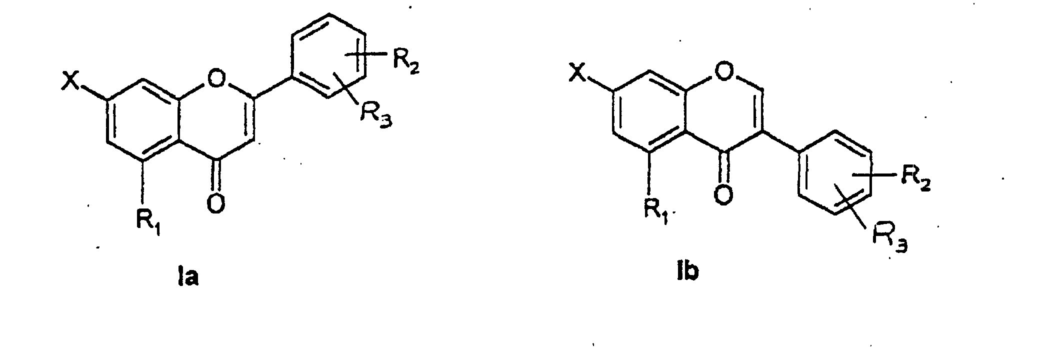 dans lesquelles X représente un groupe de formule -COOR, ou -PO(OR)₂, R représente un atome d'hydrogène ou de métal alcalin ou alcalino-terreux, ou un groupe alkyle inférieur linéaire ou ramifié de 1 à 5 atomes de carbone, R₁ représente un groupe hydroxy, un groupe alkoxy inférieur linéaire ou ramifié comportant 1 à 5 atomes de carbone ou un groupe acyloxy comportant 1 à 5 atomes de carbone, R₂ et R₃, identiques ou différents, représentent un atome d'hydrogène ou d'halogène, ou un groupe trifluorométhyle, un groupe trichlorométhyle, un groupe hydroxy, un groupe alkoxy linéaire ou ramifié de 1 à 5 atomes de carbone ou un groupe acyloxy comportant 1 à 5 atomes de carbone, ou R₂ et R₃ peuvent s'associer pour former un groupe alkylène dioxy.

Dans les formules (Ia) et (Ib) ci-dessus, X représente de préférence un groupe acide carboxylique ou phosphorique ou un ester dans lequel R est un groupe méthyle ou éthyle, R₁ représente de préférence un groupe hydroxy, acétoxy ou méthoxy.

R₂ représente de préférence un atome d'hydrogène, de chlore ou de fluor, un groupe trifluorométhyle, un groupe carboxy, ou un groupe alcoxy, et en particulier un groupe méthoxy et R₃ est de préférence un atome d'hydrogène. Le substituant représenté par R₂ est situé de préférence en position 4' du noyau phényle. R₂ et R₃ peuvent s'associer pour former ensemble un groupe éthylène dioxy ou méthylène dioxy.

Quand le substituant X est dérivé de l'acide carboxylique, il représente de préférence le groupe -COOH ou -COOCH₃. Quand il est un dérivé de l'acide phosphorique, il représente de préférence un groupe -PO(OH)₂ ou -PO(OCH₃)₂.

Les dérivés de l'invention peuvent notamment se présenter sous forme de sels d'acide carboxylique ayant l'une ou l'autre des formules (Ia) et (Ib) ci-dessus où X est un groupe -COOR, R étant plus particulièrement un atome de sodium ou de potassium.

Les nouveaux dérivés de flavones représentés par la formule (Ia) ci-dessus peuvent être préparés à partir des 5,7-dihydroxy-flavones représentées par la formule générale (IIa) ci-dessous 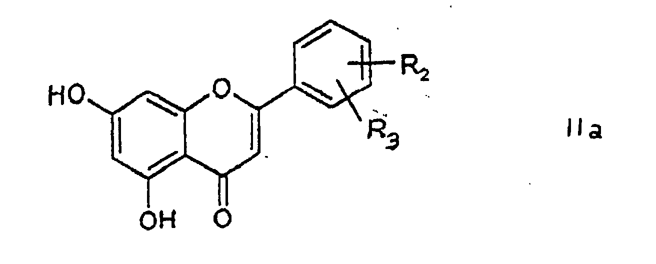 dans laquelle les substituants R₂ et R₃ ont la même signification que dans la formule (Ia),
par réaction avec un anhydride d'acide trifluoroalcanesulfonique en présence d'une base non protique dans un solvant approprié, puis action d'un chlorure ou d'un anhydride d'acide en présence d'une base non protique, pour former les dérivés de formule (IIIa) ci-après 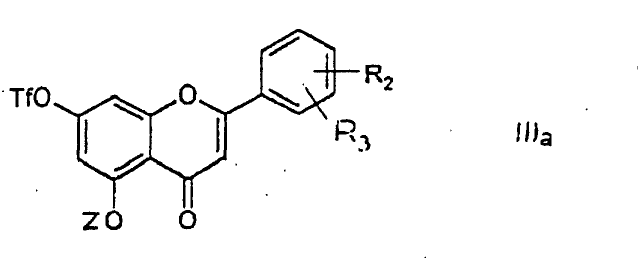 dans laquelle Z est un groupe pivaloyle, méthoxyméthyle ou trialkyle silyle, puis on effectue une carbonylation en présence d'un catalyseur à base de palladium, un ligand phosphoré et un alcool, dans un solvant approprié, et on élimine les groupements protecteurs en position 5 et 7.

La déprotection peut être faite de manière connue, en fonction des groupements protecteurs utilisés. Par exemple la déprotection peut se faire, en milieu basique pour les fonctions esters, en présence de fluorure de potassium ou de tétrabutylammonium pour un ester de triméthylsilyle, ou en milieu acide pour un groupe méthoxyméthyle.

Les dérivés d'isoflavones de formule (Ib) sont préparés de manière analogue à partir des 5,7-dihydroxy-isoflavones correspondantes représentées par la formule (IIb) ci-après, 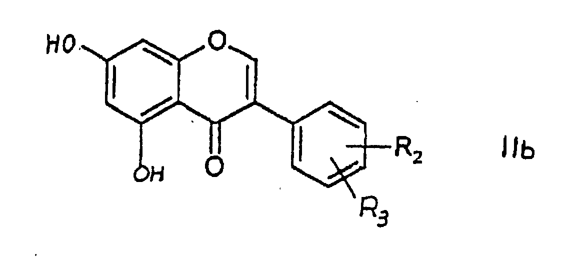 dans laquelle les substituants R₂ et R₃ ont la même signification que dans la formule (Ib),
pour obtenir l'intermédiaire de formule (IIIb) 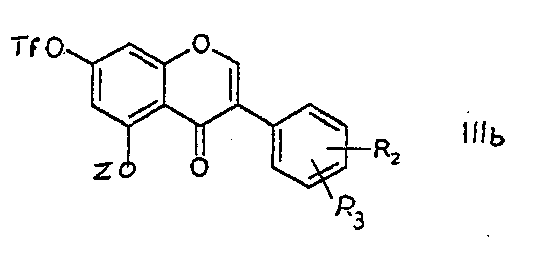 dans laquelle Z, R₂ et R₃ ont la même signification que ci-dessus, puis traitement par le même schéma réactionnel.

La réaction de l'anhydride d'acide trifluoroalcane sulfonique sur la dihydroxyflavone de formule (II) se fait en présence d'une base non protique qui peut être avantageusement choisie parmi la pyridine et la triéthylamine, et elle s'effectue de préférence dans un solvant tel que le dichlorométhane.

L'anhydride d'acide trifluoroalcane sulfonique utilisé dans le procédé suivant l'invention est de préférence l'anhydride d'acide trifluorométhanesulfonique. Le chlorure utilisé dans la réaction de préparation des dérivés de formule (IIIa) peut être choisi parmi le chlorure de pivaloyle, le chlorure de méthoxyméthyle, ou encore un chlorure de trialkylsilyle, en présence d'une base non protique telle que la pyridine.

Le catalyseur à base de palladium utilisé dans l'étape de carbonylation du procédé suivant l'invention peut être par exemple Pd(OAc)₂ en présence de monoxyde de carbone. Le dérivé phosphoré peut être par exemple le 1,3-bis(diphénylphosphino)-propane, et l'alcool peut être par exemple le méthanol ou le 2-triméthylsilyl-éthanol. Le solvant utilisé dans cette étape est de préférence le DMF ou le DMSO.

On peut observer que les flavones de départ représentées par la formule générale (IIa) pourraient servir à la préparation de 3,5,7-trihydroxy-flavones dont le groupe hydroxy en position 7 se comporterait comme le groupe correspondant des 5,7-dihydroxy-flavones, tandis que le groupe hydroxy en position 3 se comporterait comme le groupe en position 5. Ces trihydroxy-flavones peuvent être préparées à partir des dihydroxy-flavones correspondantes comportant un atome d'hydrogène en position 3, par action du diméthyldioxirane dans l'acétone, suivant la méthode de W. Adam et al. J. Org. Chem. (1991) 56, 7292-7297, ou par action du diacétate d'iodosyl phényle suivant la méthode de R.M. Moriarty et al. J. Heterocycl. Chem. (1985) 22, 583. Par contre, en série isoflavone, seuls les dérivés 5,7-dihydroxy sont stables, au contraire des 2,5,7-trihydroxy-flavones, très instables.

Les expérimentations réalisées sur les dérivés de la présente invention ont mis en évidence d'intéressantes propriétés pharmacologiques, et en particulier un important effet inhibiteur de la production de cytokines inflammatoires impliquées dans la maladie rhumatismale. Ces résultats permettent d'envisager leur utilisation dans le traitement de l'arthrose et de la polyarthrite rhumatoïde, et tour particulièrement de l'arthrose.

De plus, les essais effectués ont montré que les dérivés de carboxy-7-flavone et carboxy-7-isoflavone suivant la présente invention sont potentiellement plus actifs que les anti-inflammatoires de référence, tels que les dérivés de rhéine, en particulier la diacérhéine (la rhéine étant le métabolite actif de la diacérhéine), couramment utilisés en thérapeutique humaine dans le traitement de l'arthrose. Par contre, ils ne présentent pas les effets secondaires laxatifs inhérents à ces médicaments connus. Ces résultats sont sans doute dus au fait que les dérivés de flavones ne possèdent pas le noyau quinonique considéré comme responsable de l'effet laxatif, mais conservent les propriété inhibitrices vis-à-vis de l'interleukine-1 et d'autres cytokines.

L'activité inhibitrice de la sécrétion de cytokines inflammatoires des composés de l'invention a été testée in vitro, plus particulièrement sur la sécrétion de l'IL-1 (interleukine-1) ainsi que sur l'IL-6 et le TNFα (facteur de nécrose tumorale), qui sont les principales cytokines liées au processus arthrosique.

Les tests sur ces trois cytokines ont été effectués sur les cellules PBMC (Peripheral Blood Mononuclear Cells) selon la méthode de Schindler (R. Schindler et al., Blood (1990) 75, 40-47) en utilisant le cycloheximide (IL-1) et la dexaméthasone (IL-6, TNFα) comme produits de référence.

L'effet des dérivés de l'invention sur la sécrétion de prostaglandines (PGE₂) qui augmentent l'inflammation articulaire, et de leukotriène (LTB₄) a été mesuré sur des cellules HL-60 différenciées en utilisant les méthodes de Honda (M. Honda et al., Diabètes Res. (1990) 14, 43-46) et de Bennett (C.F. Bennett et al., Biochem. J. (1993) 289, 33-39), respectivement, en utilisant comme produits de référence l'indométhacine et l'acide nordihydroguaiarétique.

L'étude de la sécrétion d'oxygène, permettant d'évaluer l'état de stress de la cellule, a été faite sur des cellules HL-60 selon la méthode de Lorico (A. Lorico et al., Biochem. Pharmacol. (1986) 35, 2443-2445).

Des tests ont aussi été effectués sur la stimulation de la NO synthase, étant donné que l'interleukine-1 augmente la production de NO par stimulation de la NO synthase (NOS). Ces tests ont été effectués suivant la méthode de Tayeh (M.A. Tayeh et al., J. Biol. Chem., (1989) 264, 19654-19658).

Les résultats des essais effectués avec les dérivés de flavone de l'invention décrits dans les Exemples 1, 4 et 6, ci-dessus, sont regroupés au tableau ci-après. Les essais ont été réalisés en double, à des concentrations de 1 et 10 µM. Les résultats sont exprimés en pourcentage d'inhibition.

### Résultats

| Exemple | µM | IL1β | IL6 | TNFα |
|---|---|---|---|---|
| 1 | 10 1 | 96 15 | 98 50 | 32 61 |
| 4 | 1 | <10 | 56 | nd |
| 6 | 10 | 72 | 100 | nd |
| nd : résultat non disponible (test non réalisé). | | | | |

Ces résultats montrent que les dérivés de flavone suivant la présente invention exercent des effets inhibiteurs significatifs sur l'IL-1 et l'IL-6. Le dérivé de l'Exemple 4 présente de plus la particularité de stimuler la sécrétion de prostaglandines. Les pourcentages d'inhibition sont très élevés par rapport à ceux observés avec des anti-inflammatoires anti-arthrosiques de référence que sont la rhéine et la diacérhéine.

Les études toxicologiques effectuées ont montré une faible toxicité des dérivés de l'invention aux doses normalement utilisées dans les traitements.

Les propriétés pharmacologiques des dérivés de flavones et d'isoflavones selon la présente invention, jointes à leur faible toxicité, montrent qu'ils peuvent être avantageusement utilisés pour le traitement des maladies rhumatismales et tout particulièrement de l'arthrose. Ils peuvent être présentés sous forme d'esters ou de sels, en mélange, le cas échéant, avec des supports ou excipients pharmaceutiquement acceptables.

Les dérivés de flavones conformes à la présente invention peuvent être administrés sous les formes usuelles de la technique pharmaceutique, et par exemple, sous forme de comprimés, gélules, capsules, solutés injectables, solutés buvables, gels transdermiques, etc, adaptées à la voie d'administration choisie, c'est-à-dire généralement par voie orale, parentérale ou transdermique. La posologie utile est adaptée en fonction de la sévérité de l'affection à traiter, l'âge et le poids du patient ainsi que le mode d'administration retenu. Les doses unitaires sont généralement comprises entre 10 mg et 5 g par jour, en une à trois prises.

Les exemples suivants décrivent la préparation de dérivés conformes à la présente invention, donnés à titre non limitatif.

### Exemple 1

### Acide 5-hydroxy-2-phényl-4-oxo-4H-chromène-7-carboxylique

Dans une solution de 200 ml de dichlorométhane contenant 10 g de 5,7-dihydroxy-flavone, en maintenant la température à 0°C, on ajoute 12,7 ml de pyridine puis 6,6 ml d'anhydride trifluorométhane sulfonique. Après environ 3 h de réaction à 0°C, le mélange réactionnel est neutralisé par une solution de d'acide chlorhydrique 1N puis extrait au dichlorométhane.

Après évaporation du solvant, on récupère 13,9 g (rendement 92%) de 7-trifluorométhanesulfonyloxy-5-hydroxy-2-phényl-4-oxo-4H-chromène, sous forme de poudre blanche, dont la structure chimique est vérifiée par chromatographie et spectre infrarouge, et confirmée par RMN et spectre de masse.
Rf = 0,58 (AcOEt/EP = 30/70)
I.R. (cm⁻¹) : 1655 (C=O); 1620 (C=C); 1436 (S=O).

Au produit obtenu comme indiqué ci-dessus (330 mg) en solution dans 4 ml de pyridine à 0°C, on ajoute 0,16 ml de chlorure de pivaloyle, et on laisse réagir pendant environ 48 h en maintenant la température à 0°C.

Après séparation par chromatographie flash (éluants AcOEt/EP = 5/95 à 10/90) on obtient le 7-trifluorométhanesulfonyloxy-2-phényl-5-pivaloyloxy-4-oxo-4H-chromène (rendement 95%) sous forme de poudre blanche, présentant un point de fusion F = 126-128°C.
Rf = 0,65 (AcOEt/EP = 10/90)
I.R. (cm⁻¹) : 1752 (C=O ester); 1657 (C=O); 1614 (C=C); 1427 (S=O).

Le dérivé ci-dessus (200 mg) est ensuite mélangé à 8,7 mg de 1,3-bis(diphénylphosphino)propane et 4,7 mg de Pd(OAc)₂ sous atmosphère de monoxyde de carbone, dans un ballon tricol, et traité 0,2 ml de 2-triméthylsilyl-éthanol en présence de 0,12 ml de triéthylamine et 0,9 ml de DMSO. Le mélange est agité à 70°C pendant 3 h environ, extrait au dichlorométhane et lavé avec une solution d'acide chlorhydrique 1N.

Après séparation par chromatographie flash (éluants AcOEt/EP = 5/95 à 10/90) on obtient 181 mg de 2-phényl-5-pivaloyloxy-4-oxo-4H-chromène-7 carboxylate de 2-(triméthylsilyl)éthyle (rendement 91%) sous forme de cristaux blancs, présentant un point de fusion F = 205°C.
Rf = 0,26 (CH₂Cl₂/MeOH = 97/3).

Au carboxylate ci-dessus (180 mg) on ajoute 202 mg de fluorure de tétrabutylammonium en solution dans 2 ml de tétrahydrofurane à 0°C, puis on laisse réagir à température ambiante pendant environ 19 h, et on ajoute une solution de soude 1N (4,1 ml). Après 72 h, le mélange est hydrolysé par une solution d'acide chlorhydrique 1N, puis extrait à l'acétate d'éthyle.

L'acide 5-hydroxy-2-phényl-4-oxo-4H-chromène-7-carboxylique ainsi obtenu, après purification par recristallisation dans un mélange méthanol / chloroforme (1-9) se présente sous forme d'une poudre jaune.
Point de fusion F = 270°C (décomposition)
Rf = 0,24 (CH₂Cl₂/MeOH = 95/5)
I.R. (cm⁻¹) : 1724 (COOH) ; 1656 (C=O); 1614 (C=C).
RMN ¹H (CDCl₃/CD₃OD = 9/1) δppm : 6,74 (s, 1H, H-3); 7,40 et 7,66 (2s, 2H, H-6 et H-8); 7,48-7,51 (m, 3H, H-3' et H-4'); 7,88 (d, 2H, J=6,4Hz, H-2').
RMN ¹³C (DMSO) δppm : 106,4-108,9-111,3 et 112,8 (C-3, C-8, C-6 et C-4a); 127,1 (C-2'); 129,5 (C-3'); 130,6 (C-1'); 132,9 (C-4'); 137,6 (C-7); 156,0-160,1-165,1 et 166,1 (C-5, C-2, C-8a et COOH); 183,3 (C-4).

### Exemple 2

### 7-diméthoxyphosphoryl-5-hydroxy-2-phényl-4-oxo-4H-chromène

Le 7-trifluorométhanesulfonyloxy-2-phényl-5-pivaloyloxy-4-oxo-4H-chromène (500 mg), intermédiaire de synthèse obtenu dans l'Exemple 1, est traité par 0,12 ml de diméthylphosphite en présence de 0,24 ml de di-isopropyléthylamine et de 61 mg de tétrakis-(triphénylphosphine)palladium en solution dans 2,5 ml d'acétonitrile.

Après chauffage à 70°C pendant 7 h, le mélange réactionnel est neutralisé par une solution d'acide chlorhydrique 1N, puis extrait au dichlorométhane.

Après séparation par chromatographie flash (éluants AcOEt/EP = 5/95 à 50/50) on obtient 383 mg de 7-diméthoxyphosphoryl-2-phényl-5-pivaloyloxy-4-oxo-4H-chromène (rendement 84%) sous forme de poudre blanche présentant un point de fusion F = 165-167°C.

Le dérivé ci-dessus est ajouté à une solution de soude (5 ml), d'eau (10 ml) et de méthanol (10 ml). Après 23 h de réaction à température ambiante, le mélange réactionnel est acidifié par une solution d'acide chlorhydrique 1N. Le précipité formé est lavé à l'eau. Après une recristallisation dans un mélange méthanol / chloroforme, le 7-diméthoxyphosphoryl-5-hydroxy-2-phényl-4-oxo-4H-chromène est obtenu avec un rendement de 90% sous forme de précipité jaune.
Point de fusion F = 219°C
Rf = 0,25 (CH₂Cl₂/MeOH = 70/30)

### Exemple 3

### 7-dihydroxyphosphoryl-5-hydroxy-2-phényl-4-oxo-4H-chromène

On ajoute 0,55 ml de bromotriméthylsilane au produit de l'Exemple 2 en solution dans 13 ml de dichlorométhane. Après 21 h de réaction à température ambiante, le solvant est évaporé.

Après recristallisation dans un mélange éthanol / chloroforme, on obtient le 7-dihydroxyphosphoryl-5-hydroxy-2-phényl-4-oxo-4H-chromène sous forme de poudre jaune, avec un rendement de 68%.
Point de fusion F = 293-296°C (décomposition)
I.R. (cm⁻¹) : 1655 (C=O); 1616 (C=C); 1261 (P=O).
RMN ¹H (DMSO) δppm : 6,98 et 7,44 (2s, 2H, J=13,5Hz, H-6 et H-8); 7,16 (s, 1H, H-3); 7,59-7,65 (m, 3H, H-3' et H-4'); 8,15 (d, 2H, J=6,9Hz, H-2'); 12,63 (s, OH).
RMN ¹³C (DMSO) δppm : 107,7 (C-3); 110,8 (C-4a); 111,3 et 113,9 (J=9Hz, C-6 et C-8); 128,4 (C-2'); 130,9 (C-3'); 132,1 (C-1'); 134,2 (C-4'); 156,9 et 161,1 (J=20Hz, C-5, C-2, C-8a); 166,3 (C-2); 183,2 (C-4).

### Exemple 4

### Acide 5-hydroxy-2-(4'-méthoxyphényl)-4-oxo-4H-chromène-7-carboxylique

On procède comme indiqué dans l'Exemple 1, en remplaçant la 5,7-dihydroxy-flavone de départ par la (4'-méthoxy)-5,7-dihydroxy-flavone, dans les mêmes conditions opératoires.

On obtient ainsi le 2-(4'-méthoxyphényl)-5-pivaloyloxy-4-oxo-4H-chromène-7 carboxylate de 2-(triméthylsilyl)éthyle, qui est traité dans les mêmes conditions que dans l'Exemple 1 pour procurer l'acide 5-hydroxy-2-(4'-méthoxyphényl)-4-oxo-4H-chromène-7-carboxylique
Point de fusion F = 277-278°C
Rf = 0,53 (CH₂Cl₂/MeOH = 90/10)

### Exemple 5

### Acide 5-hydroxy-3-(4'-méthoxyphényl)-4-oxo-4H-chromène-7-carboxylique

Ce dérivé est obtenu comme dans l'Exemple précédent, en remplaçant la flavone de départ par l'isoflavone correspondante.

On obtient ainsi l'acide 5-hydroxy-3-(4'-méthoxyphényl)-4-oxo-4H-chromène-7-carboxylique.
Rf = 0,36 (CH₂Cl₂/MeOH = 90/10)
I.R. (cm⁻¹) : 1705 (COOH) ; 1653 (C=O); 1611 et 1583 (C=C).
RMN ¹H (DMSO) δppm : 3?87 (s, 3H, CH₃O-); 7,09 (s, 1H, H-3); 7,11 (d, 2H, H-6 et H-8); 8,113 (d, 2H, J=8,9Hz, H-2').
RMN ¹³C (DMSO) δppm : 55,9 (CH3) ; 104,6-108,7 et 111,1 (C-3, C-6 et C-8); 112,5 (C-4a); 114,9 (C-3'); 122,6 (C-1'); 129,1 5 (C-2'); 137,5 (C-7); 155,8-160,0-163,0-165,2 et 166,1 (C-2, C-4', C-5, C-8a et COOH); 183,0 (C-4).

### Exemple 6

### Acide 5-hydroxy-2-(4'-trifluorométhylphényl)-4-oxo-4H-chromène-7-carboxylique

On procède comme dans l'Exemple 1 en remplaçant la 5,7-dihydroxy-flavone de départ par la (4'-trifluorométhyl)-5,7-dihydroxy-flavone, dans les mêmes conditions opératoires.

On obtient ainsi l'acide 5-hydroxy-3-(4'-trifluorométhylphényl)-4-oxo-4H-chromène-7-carboxylique.
Point de fusion F = 278°C (décomposition)
Rf = 0,18 (CH₂Cl₂/MeOH = 90/10).
RMN ¹H (CDCl₃/CD₃OD = 9/1) δppm : 6,88 (s, 1H, H-3); 7,49 et 7,74 (2d, 2H, J=1,2Hz, H-6 et H-8); 7,82 (d, 2H, J=8,3Hz, H-3'); 8,09 (d, 2H, J=8,2Hz, H-2').
RMN ¹³C (DMSO) δppm : 108,5-109,6-112,1 et 113,4 (C-3, C-4a, C-6 et C-8); 124,7 (q, J=271,0Hz, CF₃); 126,97 (q, J=3,6Hz, C-3'); 128,6 (C-2'); 132,8 (q, J=32,0Hz, C-4'); 135,2 (C-7); 138,8 (C-1); 156,6-160,7-163,9 et 166,8 (C-2, C-5, C-8a et COOH); 184,0 (C-4).

## Revendications

1. Dérivés de flavones et d'isoflavones représentés par les formules générales (Ia) et (Ib) ci-après : 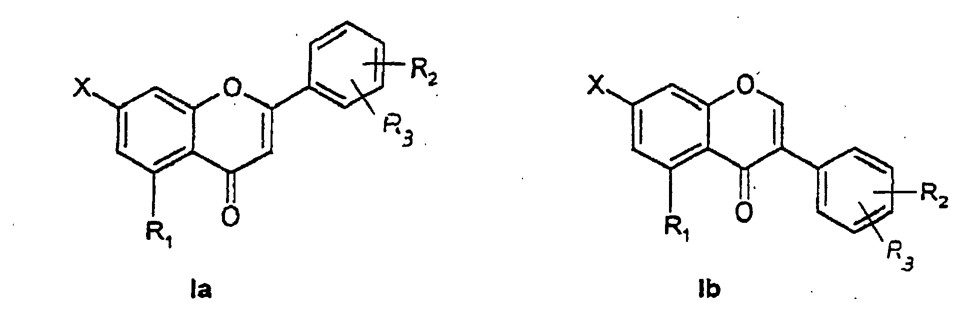 dans lesquelles X représente un groupe de formule -COOR, ou -PO(OR)₂, R représente un atome d'hydrogène ou de métal alcalin ou alcalino-terreux, ou un groupe alkyle inférieur linéaire ou ramifié de 1 à 5 atomes de carbone, R₁ représente un groupe hydroxy, un groupe alkoxy inférieur linéaire ou ramifié comportant 1 à 5 atomes de carbone ou un groupe acyloxy comportant 1 à 5 atomes de carbone, R₂ et. R₃, identiques ou différents, représentent un atome d'hydrogène ou d'halogène, ou un groupe trifluorométhyle, un groupe trichlorométhyle, un groupe hydroxy, un groupe alkoxy linéaire ou ramifié de 1 à 5 atomes de carbone ou un groupe acyloxy comportant 1 à 5 atomes de carbone, ou R₂ et R₃ peuvent s'associer pour former un groupe alkylène dioxy.

2. Dérivés selon la revendication 1, **caractérisés en ce que** X représente un groupe acide carboxylique ou phosphorique ou un ester dans lequel R est un groupe méthyle, et R₁ représente un groupe hydroxy, acétoxy ou méthoxy,

3. Dérivés selon l'une quelconque des revendications 1 et 2, **caractérisés en ce que** R₂ représente un atome d'hydrogène, un atome de fluor, un groupe trifluorométhyle, un groupe carboxy, ou un groupe alkoxy, et R₃ est un atome d'hydrogène, ou R₂ et R₃ forment ensemble un groupe éthylène dioxy ou méthylène dioxy.

4. Dérivés selon l'une quelconque des revendications 2 et 3, **caractérisés en ce que** X est un groupe -COOH, R₁ est un groupe hydroxy et R₂ représente un atome d'hydrogène, un groupe trifluorométhyle ou un groupe méthoxy situé en position 4' du noyau phényle.

5. Dérivés selon la revendication 2, **caractérisés en ce que** X est un groupe -COOR, où R est atome de sodium ou de potassium.

6. Dérivés selon l'une quelconque des revendications précédentes, **caractérisés en ce qu'**ils sont choisis parmi l'acide 5-hydroxy-2-phényl-4-oxo-4H-chromène-7-carboxylique, le 7-dihydroxyphosphoryl-5-hydroxy-2-phényl-4-oxo-4H-chromène, l'acide 5-hydroxy-2-(4'-méthoxyphényl)-4-oxo-4H-chromène-7-carboxylique et l'acide 5-hydroxy-2-(4'-trifluorométhylphényl)-4-oxo-4H-chromène-7-carboxylique.

7. Procédé de préparation de dérivés de flavones et d'isoflavones représentés par les formules générales (Ia) et (Ib) selon la revendication 1, **caractérisé en ce que** l'on fait réagir une 5,7-dihydroxy-flavone représentée par la formule générale (IIa) ou (IIb) ci-dessous 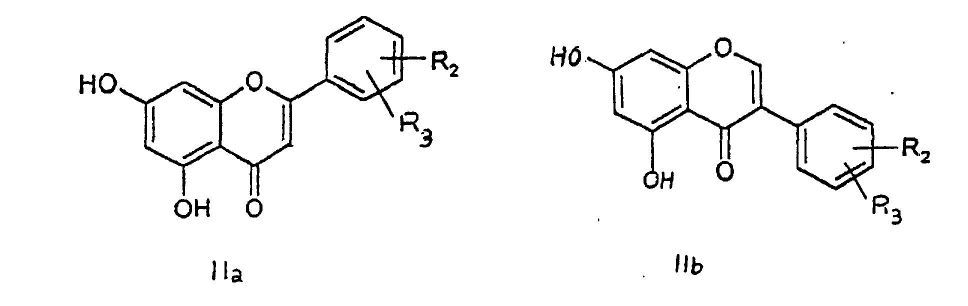 dans laquelle les substituants R₂ et R₃ ont la même signification que dans la revendication 1,
avec un anhydride d'acide trifluoroalcanesulfonique en présence d'une base non protique dans un solvant approprié, puis on fait agir un chlorure ou un anhydride d'acide en présence d'une base non protique, pour former les dérivés de formule (IIIa) ou (IIIb) ci-après 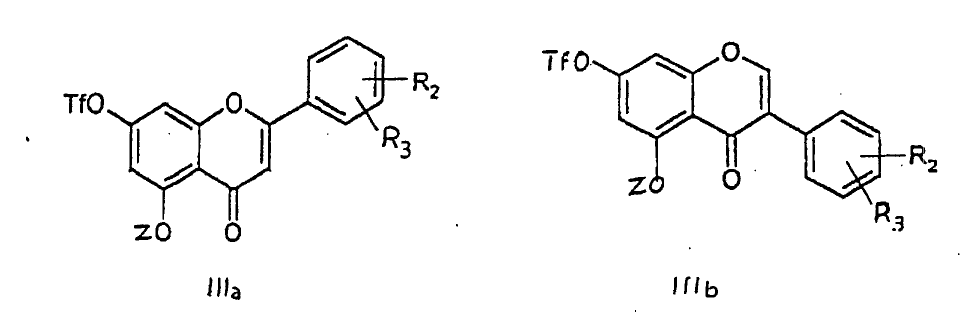 dans laquelle Z est un groupe pivaloyle, méthoxyméthyle ou trialkyle silyle, puis on effectue une carbonylation en présence d'un catalyseur à base de palladium, un ligand phosphoré et un alcool, dans un solvant approprié, et on élimine les groupements protecteurs en position 5 et 7, et, le cas échéant, en position 3.

8. Procédé selon la revendication 7,**caractérisé en ce que** le chlorure est choisi parmi le chlorure de pivaloyle, le chlorure de méthoxyméthyle, ou encore un chlorure de trialkylsilyle.

9. Procédé selon l'une quelconque des revendications 7 et 8, **caractérisé en ce que** la base non protique est choisie parmi la pyridine et la triéthylamine.

10. Procédé selon la revendication 7, **caractérisé en ce que** la carbonylation est effectuée en présence d'un catalyseur à base de palladium constitué par Pd(OAc)₂ en présence de monoxyde de carbone, le ligand phosphoré est le 1,3-bis(diphénylphosphino)propane, et l'alcool est choisi parmi le méthanol et le 2-triméthylsilyl-éthanol.

11. Procédé selon la revendication 10, **caractérisé en ce que** la réaction de carbonylation est effectuée dans un solvant choisi parmi le DMF et le DMSO.

12. Composition pharmaceutique utile en thérapeutique humaine et vétérinaire, **caractérisée en ce qu'**elle comprend un dérivé de flavone ou d'isoflavone selon l'une quelconque des revendications 1 à 6, et le cas échéant un ou plusieurs excipients ou supports pharmaceutiquement acceptables.

13. Utilisation d'un dérivé de flavone ou d'isoflavone selon la revendication 1 pour la préparation d'un médicament pour le traitement des maladies rhumatismales telles que l'arthrose ou la polyarthrite rhumatoïde.

## Patentansprüche

1. Flavon- und Isoflavonderivate der nachstehenden allgemeinen Formeln (Ia) und (Ib): 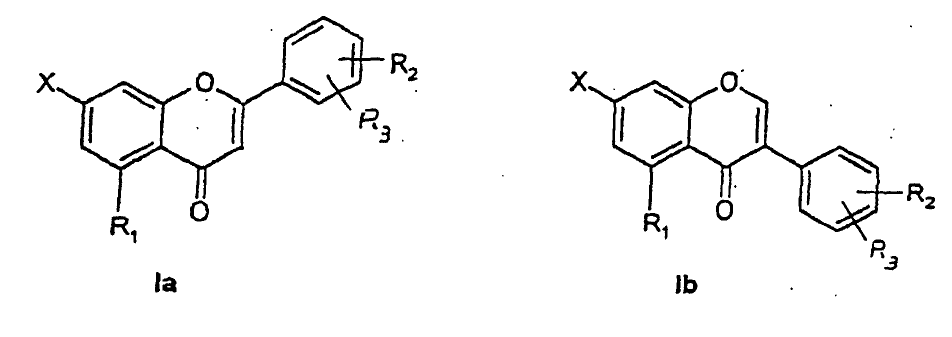 worin X für eine Gruppe der Formel -COOR oder -PO(OR)₂ steht, R für ein Wasserstoffatom oder ein Alkali- oder Erdalkalimetall oder eine unverzweigte oder verzweigte Niederalkylgruppe mit 1 bis 5 Kohlenstoffatomen steht, R₁ für eine Hydroxygruppe, eine unverzweigte oder verzweigte Niederalkoxygruppe mit 1 bis 5 Kohlenstoffatomen oder eine Acyloxygruppe mit 1 bis 5 Kohlenstoffatomen steht, R₂ und R₃, die gleich oder voneinander verschieden sind, für ein Wasserstoff- oder Halogenatom oder eine Trifluormethylgruppe, eine Trichlormethylgruppe, eine Hydroxygruppe, eine unverzweigte oder verzweigte Alkoxygruppe mit 1 bis 5 Kohlenstoffatomen oder eine Acyloxygruppe mit 1 bis 5 Kohlenstoffatomen stehen oder R₂ und R₃ zu einer Alkylendioxygruppe verbunden sein können.

2. Derivat nach Anspruch 1, **dadurch gekennzeichnet, dass** X für eine Carbon- oder Phosphorsäuregruppe oder einen Ester steht, in dem R eine Methylgruppe ist, und R₁ für eine Hydroxy-, Acetoxy- oder Methoxygruppe steht.

3. Derivat nach einem der Ansprüche 1 bis 2, **dadurch gekennzeichnet, dass** R₂ für ein Wasserstoffatom, ein Fluoratom, eine Trifluormethylgruppe, eine Carboxygruppe oder eine Alkoxygruppe steht und R₃ ein Wasserstoffatom ist oder R₂ und R₃ gemeinsam eine Ethylendioxy- oder Methylendioxygruppe bilden.

4. Derivat nach einem der Ansprüche 2 und 3, **dadurch gekennzeichnet, dass** X eine Gruppe -COOH ist, R₁ eine Hydroxygruppe ist und R₂ für ein Wasserstoffatom, eine Trifluormethylgruppe oder eine Methoxygruppe steht, die sich an Position 4' des Phenylkerns befindet.

5. Derivat nach Anspruch 2, **dadurch gekennzeichnet, dass** X eine Gruppe -COOR ist, wobei R ein Natrium- oder Kaliumatom ist.

6. Derivat nach einem der vorangegangenen Ansprüche, **dadurch gekennzeichnet, dass** es aus 5-Hydroxy-2-phenyl-4-oxo-4H-chromen-7-carbonsäure, 7-Dihydroxyphosphoryl-5-hydroxy-2-phenyl-4-oxo-4H-chromensäure, 5-Hydroxy-2-(4'-methoxyphenyl)-4-oxo-4H-chromen-7-carbonäsure und 5-Hydroxy-2-(4'-trifluormethylphenyl)-4-oxo-4Hchromen-7-carbonsäure ausgewählt ist.

7. Verfahren zur Herstellung von Flavon- und Isoflavonderivaten der allgemeinen Formeln (Ia) und (Ib) nach Anspruch 1, **dadurch gekennzeichnet, dass** ein 5,7-Dihydroxyflavon der nachstehenden allgemeinen Formel (IIa) oder (IIb) 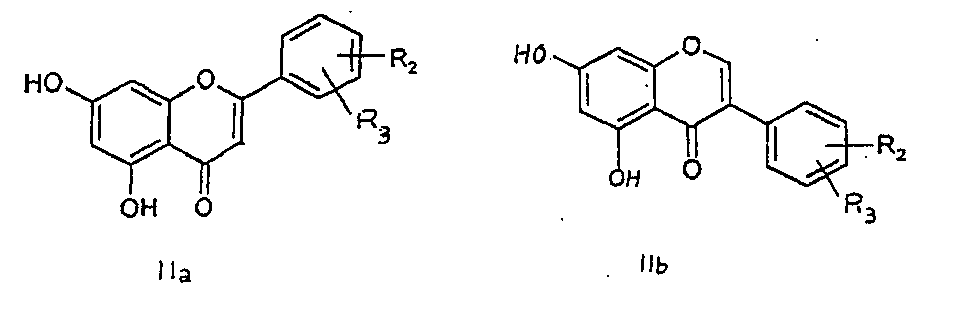 worin die Substituenten R₂ und R₃ die gleiche Bedeutung wie in Anspruch 1 haben, mit einem Trifluoralkansulfonsäureanhydrid in Gegenwart einen aprotischen Base in einem geeigneten Lösungsmittel umgesetzt wird, dann ein Säurechlorid oder ein Säureanhydrid in Gegenwart einer aprotischen Base einwirken gelassen wird, um die Derivate der nachstehenden Formel (IIIa) oder (IIIb) zu bilden: 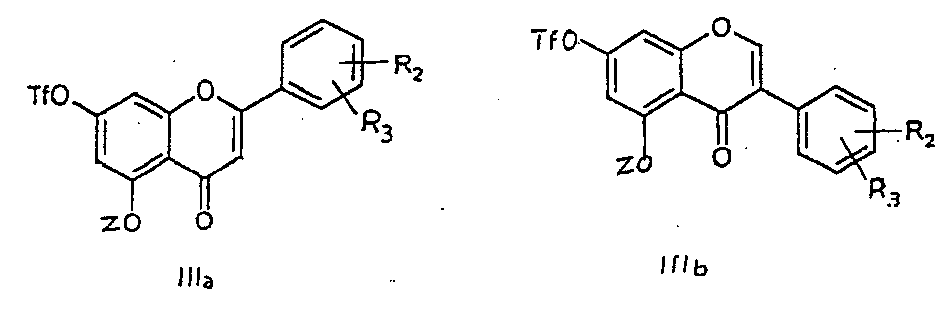 worin Z eine Pivaloyl-, Methoxymethyl- oder Trialkylsilylgruppe ist, dann eine Carbonylierung in Gegenwart eines Katalysators auf Palladium-Basis, eines Phosphorliganden und eines Alkohols in einem geeigneten Lösungsmittel durchgeführt wird und die Schutzgruppen an Position 5 und 7 und gegebenenfalls an Position 3 entfernt werden.

8. Verfahren nach Anspruch 7, **dadurch gekennzeichnet, dass** das Chlorid aus Pivaloylchlorid, Methoxymethylchlorid oder aber einem Trialkylsilylchlorid ausgewählt ist.

9. Verfahren nach einem der Ansprüche 7 und 8, **dadurch gekennzeichnet, dass** die aprotische Base aus Pyridin und Triethylamin ausgewählt ist.

10. Verfahren nach Anspruch 7, **dadurch gekennzeichnet, dass** die Carbonylierung in Gegenwart eines Katalysators auf Palladiumbasis, der aus Pd(OAc)₂ besteht, in Gegenwart von Kohlenmonoxid durchgeführt wird, der Phosphorligand 1,3-Bis(diphenylphosphino)propan ist und der Alkohol aus Methanol und 2-Trimethylsilylethanol ausgewählt ist.

11. Verfahren nach Anspruch 10, **dadurch gekennzeichnet, dass** die Carbonylierungsreaktion in einem Lösungsmittel durchgeführt wird, das aus DMF und DMSO ausgewählt ist.

12. Pharmazeutische Zusammensetzung, die zur Therapie von Mensch und Tier geeignet ist, **dadurch gekennzeichnet, dass** sie ein Flavon- oder Isoflavonderivat nach einem der Ansprüche 1 bis 6 und gegebenenfalls einen oder mehrere pharmazeutisch annehmbare Exzipienten oder Träger umfasst.

13. Verwendung eines Flavon- oder Isoflavonderivats nach Anspruch 1 zur Herstellung eines Medikaments zur Behandlung rheumatischer Erkrankungen wie Arthrose oder rheumatischer Polyarthritis.

## Claims

1. Flavone and isoflavone derivatives represented by the following general formulae (Ia) and (Ib): 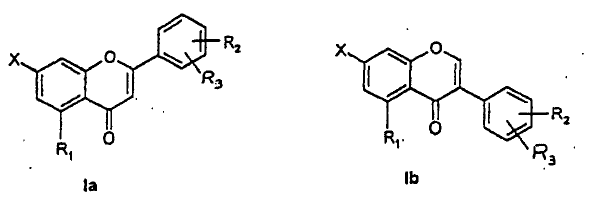 in which X represents a group of formula -COOR or -PO(OR)₂, R represents a hydrogen atom or an alkali metal or alkaline earth metal atom, or a linear or branched lower alkyl group comprising 1 to 5 carbon atoms, R₁ represents a hydroxyl group, a linear or branched lower alkoxy group comprising 1 to 5 carbon atoms or an acyloxy group comprising 1 to 5 carbon atoms, R₂ and R₃, which are identical or different, represent a hydrogen or halogen atom or a trifluoromethyl group, a trichloromethyl group, a hydroxyl group, a linear or branched alkoxy group comprising 1 to 5 carbon atoms or an acyloxy group comprising 1 to 5 carbon atoms, or R₂ and R₃ can combine to form an alkylenedioxy group.

2. Derivatives according to Claim 1, **characterized in that** X represents a carboxylic or phosphoric acid group or an ester in which R is a methyl group, and R₁ represents a hydroxyl, acetoxy or methoxy group.

3. Derivatives according to either one of Claims 1 and 2, **characterized in that** R₂ represents a hydrogen atom, a fluorine atom, a trifluoromethyl group, a carboxyl group or an alkoxy group and R₃ is a hydrogen atom, or R₂ and R₃ together form an ethylenedioxy or methylenedioxy group.

4. Derivatives according to either one of Claims 2 and 3, **characterized in that** X is a -COOH group, R₁ is a hydroxyl group and R₂ represents a hydrogen atom, a trifluoromethyl group or a methoxy group situated in the 4' position of the phenyl ring.

5. Derivatives according to Claim 2, **characterized in that** X is a -COOR group where R is a sodium atom or a potassium atom.

6. Derivatives according to any one of the preceding claims, **characterized in that** they are chosen from 5-hydroxy-2-phenyl-4-oxo-4H-chromene-7-carboxylic acid, 7-dihydroxyphosphoryl-5-hydroxy-2-phenyl-4-oxo-4H-chromene, 5-hydroxy-2-(4'-methoxyphenyl)-4-oxo-4H-chromene-7-carboxylic acid and 5-hydroxy-2-(4'-trifluoromethylphenyl)-4-oxo-4H-chromene-7-carboxylic acid.

7. Process for the preparation of flavone and. isoflavone derivatives represented by the general formulae (Ia) and (Ib) according to Claim 1, **characterized in that** a 5,7-dihydroxyflavone, represented by the following general formula (IIa) or (IIb) 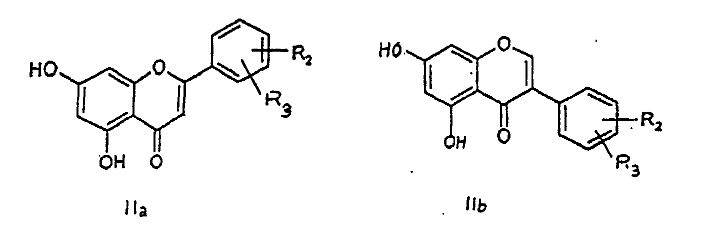 in which the R₂ and R₃ substituents have the same meanings as in Claim 1,
is reacted with a trifluoroalkanesulfonic anhydride in the presence of a nonprotic base in an appropriate solvent, then an acid chloride or an acid anhydride is reacted in the presence of a nonprotic base, to form the derivatives of formula (IIIa) or (IIIb) below 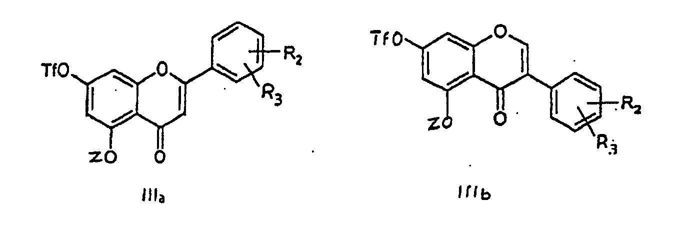 in which Z is a pivaloyl, methoxymethyl or trialkylsilyl group, then a carbonylation is carried out in the presence of a palladium-based catalyst, a phosphorus-comprising ligand and an alcohol, in an appropriate solvent, and the protective groups in the 5 and 7 position [sic], and, if appropriate, in the 3 position, are removed.

8. Process according to Claim 7, **characterized in that** the chloride is chosen from pivaloyl chloride, methoxymethyl chloride or a trialkylsilyl chloride.

9. Process according to either of Claims 7 and 8, **characterized in that** the nonprotic base is chosen from pyridine and triethylamine.

10. Process according to Claim 7, **characterized in that** the carbonylation is carried out in the presence of a palladium-based catalyst composed of Pd(OAc)₂ in the presence of carbon monoxide, the phosphorus-comprising ligand is 1,3-bis(diphenylphosphino)propane and the alcohol is chosen from methanol and 2-(trimethylsilyl)ethanol.

11. Process according to Claim 10, **characterized in that** the carbonylation reaction is carried out in a solvent chosen from DMF and DMSO.

12. Pharmaceutical composition of use in human and veterinary therapy, **characterized in that** it comprises a flavone or isoflavone derivative according to any one of Claims 1 to 6 and, if appropriate, one or more pharmaceutically acceptable excipients or carriers.

13. Use of a flavone or isoflavone derivative according to Claim 1 in the preparation of a medicament for the treatment of rheumatic diseases, such as osteoarthritis or rheumatoid arthritis.
